# EUROPÄISCHE PATENTSCHRIFT

(11) **EP 2 481 385 B1**
(45) Veröffentlichungstag und Bekanntmachung des Hinweises auf die Patenterteilung: **01.10.2014**
(21) Anmeldenummer: 12165872.8
(22) Anmeldetag: 01.08.2008
(51) Int. Cl.: A61F 13/15, A61L 15/60, A61L 15/42

(54) **Wundpflegeartikel mit absorbierender Hülle**
Wound care article having an absorbent shell
Article pour le soin des plaies avec enveloppe absorbante

(30) Priorität: 03.08.2007 DE 102007036758
(43) Veröffentlichungstag der Anmeldung: 01.08.2012
(62) Teilanmeldung aus: 08786783.4
(73) Patentinhaber: Riesinger, Birgit, 48149 Münster (DE)
(72) Erfinder: Riesinger, Birgit, 48149 Münster (DE)
(74) Vertreter: Michalski Hüttermann & Partner Patentanwälte mbB

(56) Entgegenhaltungen:
- WO-A-01/52780
- WO-A-03/094813
- WO-A-2007/051599
- WO-A1-01/15644
- US-A- 6 103 358

## Beschreibung

Die Erfindung betrifft einen Wundpflegeartikel, der sich aus wenigstens einem die flüssigen Wundexsudate absorbierenden Körper und wenigstens einer den Körper wenigstens teilweise umgebenden Hülle zusammensetzt.

Exsudate sind über die entzündlichen Prozesse des Wundödems vom Blutplasma abgeleitete Wundflüssigkeiten. So wie das Blut für den Transport von Nährstoffen und anderen Botenstoffen und damit für die Versorgung verschiedener Teile des Körpers verantwortlich ist, dient das Exsudat auf ganz ähnliche Weise der Versorgung des Wundbettes und der darin ablaufenden Heilungsprozesse. Um dieser Vielzahl an Funktionen gerecht zu werden, enthält es ein breites Spektrum an Komponenten, woraus ein spezifisches Gewicht resultiert, das leicht oberhalb dessen von Wasser liegt. Darin unterscheidet es sich auch vom Transsudat, welches eher von nicht-entzündlichen Prozessen abgeleitet ist und ein geringeres spezifisches Gewicht mit einem geringen Zell- und Proteingehalt aufweist. Neben der Bereitstellung von Nährstoffen für die Fibroblasten und Epithelzellen koordiniert das Exsudat die verschiedenen Prozesse der Wundheilung zeitlich und räumlich durch seinen hohen Gehalt an Wachstumsfaktoren und Zytokinen. Diese werden vor allem durch Thrombozyten, Keratinozyten, Makrophagen und Fibroblasten gebildet. Sie beeinflussen die Motilität, Migration und Proliferation der verschiedenen an der Wundheilung beteiligten Zellen. So wird das Einwandern von Zellen in den Wundgrund ebenso gefördert wie die Versorgung des neugebildeten Granulationsgewebes durch die Angiogenese. Auch die Wundreinigung wird durch das Exsudat unterstützt. Es enthält verschiedene Serin-, Cystein- und Aspartatproteasen sowie Matrix-Metalloproteasen,die in ihrer Aktivität streng reguliert irreversibel geschädigtes Gewebe abbauen und somit das Wundbett für die nachfolgenden Phasen der Heilung vorbereiten.

Bestandteile des physiologischen Exsudats sind insbesondere Salze, Glucose, Zytokine und Wachstumsfaktoren, Plasmaproteine, Proteasen (insbesondere Matrix-Metalloproteasen), Granulozyten und Makrophagen.

Kommt es nicht innerhalb einiger Wochen zu einer deutlichen Progression des Wundheilungsverlaufs entsprechend der verschiedenen Phasen der Wundheilung, so spricht man von einer chronischen Wunde. Dabei betrachtet man jedoch bereits länger andauernde exsudative Phasen als Komplikation und spricht von einer pathologischen Exsudation, welche zu einer Chronifizierung der Wunde beitragen kann. Die zugrundeliegenden Ursachen sind meist komplex und können durchaus auch systemischer Natur sein. Es überrascht jedoch aufgrund der zuvor erläuterten Bedeutung des Exsudats für die Wundheilung nicht, dass sich Komplikationen der Wundheilung in einer deutlich veränderten Zusammensetzung und Wirkung des Exsudats widerspiegeln.

Unter anderem durch eine Konzentrationsverschiebung der einzelnen Bestandteile des Exsudats verliert das normalerweise heilungsfördemde Exsudat bei chronischen Wunden seine positive Wirkung. Insbesondere der Gehalt an inflammatorischen Zytokinen und Proteasen ist in pathologischem Exsudat signifikant erhöht. Der Gehalt an Wachstumsfaktoren ist dagegen verringert. Ein besonders gravierender Unterschied ergibt sich hinsichtlich der Aktivität der zuvor angesprochenen Matrix-Metalloproteasen. Neben der Vorbereitung des Wundbetts sind sie auch beim späteren Umbau des Granulations- zum Narbengewebe beteiligt. Diese Enzyme werden normalerweise als inaktives Präenzym gebildet und in ihrer Aktivierung durch entsprechende Inhibitoren reguliert (tissue inhibitors of metalloproteases, TIMPs), welche gleichzeitig selbst eine positive Wirkung auf das Zellwachstum haben. Im chronischen Exsudat scheint aufgrund von Störungen in diesem Regulationssystem die Aktivität der Proteasen erhöht, was möglicherweise zu einer aktiven Wundregression beiträgt. Wirkungen des pathologischen Exsudats. Das pathologische Exsudat ist hinsichtlich des Gehalts seiner Komponenten aus dem der Wundprogression förderlichen Gleichgewicht geraten. Daraus ergeben sich verschiedene Komplikationen, die zur weiteren Verschlechterung und Chronifizierung der Wunde beitragen.
1. Hemmung der Zellproliferation: Das physiologische Exsudat unterstützt aufgrund seiner Zusammensetzung die zur Heilung notwendige Proliferation der verschiedenen Zellen. Pathologisches Exsudat ist dazu nicht in der Lage.
2. Aktive Schädigung des Wundrandes: Die nicht unmittelbar geschädigte Haut am Rand der Wunde wird durch das physiologische Exsudat angegriffen und die Wunde so aktiv vergrößert.
3. Degradation des Fibronektins: Die für die Interaktion vieler Zellen mit ihrer Umgebung notwendigen Fibronektine sind beim pathologischen Exsudat degradiert. Somit werden die Fibroblasten in ihrer für die Progression der Heilung notwendigen reparativen Funktion beeinträchtigt.
4. Veränderte Mitose der Fibroblasten: Die Zellteilung der Fibroblasten wird durch das pathologische Exsudat beeinträchtigt. Anstelle der normalerweise vorhandenen mitotisch kompetenten Zellen finden sich ruhende Zellen, was möglicherweise auf den Mangel an relevanten Wachstumsfaktoren zurückzuführen ist.

Entsprechend der schädigenden Wirkung, die durch das pathologische Exsudat auf die Wunde ausgeübt wird und damit den Prozess der Chronifizierung weiter voran treibt, ist das Entfernen des pathologischen Exsudats meist Voraussetzung für eine Progression der Wundsituation. Aus dem Stand der Technik sind hierfür Produkte bekannt.

So beschreibt die DE10059439 ebenso wie die WO03094813 sowie die WO 01/52780 der Anmelderin der vorliegenden Erfindung, eine Wundauflage zur Absorption von Wundexsudaten handeln, die superabsorbierende Polymere ("Superabsorber") enthält, und die hervorragende Eigenschaften betreffend die Aufnahme von Wundexsudaten aufweisen. Es hat sich herausgestellt, dass die Verwendung besagter Wundauflage die Heilung von chronisch exsudierenden Wunden, wie sie z.B. bei Ulcus cruris auftreten, erheblich begünstigt.

Die WO 2007/051599 der Anmelderin beschreibt eine Wundauflage zur Absorption von Wundexsudaten, die einen mit einer Hülle umgebenden Absorptionskörper aufweist, der superabsorbierende Polymere enthält. Jedoch weist dieser Absorptionskörper keine Hülle aus Weichschaumstoff, vorzugsweise Polyurethanschaum auf.

Bei dem bekannten, als Absorptionskörper bezeichneten Wundpflegeartikel handelt sich um eine Wundauflage, deren Hülle lediglich die Aufgabe hat, Wundflüssigkeiten in den im Inneren der Hülle befindlichen absorbierenden Körper weiterzuleiten. Es hat sich gezeigt, dass das Aufnahmevermögen der Wundauflage in manchen Fällen, insbesondere bei stark sezernierenden Wunden, unzureichend ist.

Aufgabe der Erfindung ist, einen verbesserten gattungsgemäßen Wundpflegeartikel zu konzipieren, der insbesondere für stark und sehr stark sezernierende bzw. exsudierende Wunden geeignet ist. Der Wundpflegeartikel soll sowohl bei der flächigen als auch bei den tiefen Wunden, wie Kavitäten, Verwendung finden.

Weitere Aufgabe der vorliegenden Erfindung ist es, einen Wundpflegeartikel zu schaffen, der einen in Bezug auf seine Gesamtfläche größeren relativen Anteil an aktiv saugender Fläche aufweist.

Diese Aufgaben werden mit den Merkmalen des vorliegenden Anspruchssatzes gelöst. So betriff die vorliegende Erfindung einen Wundpflegeartikel, bestehend aus wenigstens einem Wundflüssigkeiten absorbierenden Körper und wenigstens einer den Körper wenigstens teilweise umgebenden Hülle dadurch gekennzeichnet, dass die Hülle selbst flüssigkeitsabsorbierend ist und die Hülle aus Weichschaumstoff, vorzugsweise Polyurethanschaum, aufweist, wobei der absorbierende Körper lose im Inneren der Hülle angeordnet ist. Die Unteransprüche geben bevorzugte Ausführungsformen an. Dabei ist zu beachten, dass die genannten Bereichsangaben durchweg einschließlich der jeweiligen Grenzwerte zu verstehen sind.

Erfindungsgemäß ist ein Wundpflegeartikel der eingangs genannten Art vorgesehen, bei dem die Hülle selbst flüssigkeitsabsorbierend ist. Dadurch, dass die Hülle selbst erhebliche Mengen an Wundflüssigkeiten aufnehmen kann, erhöht sich das gesamte Aufnahmevermögen des Wundpflegeartikels. Hinzu kommt, dass die absorbierende Funktion so nahe wie möglich an die Wunde herangebracht wird.

Hierbei kann überdies vorgesehen sein, dass die Hülle ein geringeres Aufnahmevermögen aufweist als der im Inneren der Hülle angeordnete absorbierende Körper, beispielsweise in dem sie mit einem geringeren Anteil an absorbierenden Substanzen oder mit anderen absorbierenden Materialien versetzt ist.

Auf diese Weise ist gewährleistet, dass die Hülle aufgrund ihrer geringeren Saugfähigkeit stets einen trockenen Griff aufweist. Überdies weist die Hülle so eine geringere Retentionskraft und folglich eine stärkere rückfeuchtende Wirkung auf.

In einer bevorzugten Ausgestaltung ist dabei vorgesehen, dass die Wundauflage überdies eine den Wundflüssigkeiten absorbierenden Körper wenigstens teilweise umgebende Hülle aufweist, die nicht flüssigkeitsabsorbierend ist, und die
a) als Wunddistanzgittter fungiert, oder die
b) eine Wäscheschutzfunktion aufweist.

Dabei können die flüssigkeitsabsorbierende Hülle und die nicht flüssigkeitsabsorbierende Hülle Teil einer Gesamthülle sein. So kann z.B.
a) die nicht flüssigkeitsabsorbierende Hülle als Unterseite (also der Wunde zugewandte Seite) der Gesamthülle fungieren, und die flüssigkeitsabsorbierende Hülle als Oberseite (also der Wunde abgewandte Seite) der Gesamthülle fungieren, oder
b) die nicht flüssigkeitsabsorbierende Hülle als Oberseite der Gesamthülle fungieren, und die flüssigkeitsabsorbierende Hülle als Unterseite der Gesamthülle fungieren,

Variante a) ist insbesondere dann interessant, wenn es sich bei der nicht flüssigkeitsabsorbierenden Hülle um ein Wunddistanzgittter handelt.

Ein Wunddistanzgitter ist z.B. aus der DE102006017194 der Anmelderin der vorliegenden Erfindung bekannt. Selbiges besteht beispielsweise aus einem Materialabschnitt aus einem Thermoplast, aufweisend eine erste, glatte Oberfläche, eine der glatten Oberfläche abgewandte, zweite Oberfläche des Materialabschnittes und eine Vielzahl von dreidimensionalen Perforationen, deren Wände von der ersten, glatten Oberfläche beginnend jeweils in einen Randüberstand mit freier Kante auslaufen und der zweiten Oberfläche einen rauen Griff verleihen.

Die Perforationen werden z.B. mit Hilfe eines an einer Trommel angeordneten Lochsiebs im thermisch unterstützten Vakuumverfahren hergestellt. Dementsprechend weist das fertige Folienmaterial eine glatte und eine rauhe, durch die abgewinkelten Wandungen der Perforationen gebildete Oberfläche auf.

Das Wunddistanzgitter liegt auf seiner Flachseite gelegen, entweder auf den planen Flächen zwischen den Löchern der glatten Oberfläche oder auf den rauen Begrenzungen der Perforationen, da der Übergang von dem Gittermaterial zu den Löchern die Auflagefläche der rauen Oberfläche bestimmt. So kann dieses Wunddistanzgitter in zwei Orientierungen mit zwei sehr unterschiedlichen Funktionen Wundkontakt haben.

Die Aufbringung mit der glatten Seite ist besonders vorteilhaft, denn diese bestimmt die Eigenschaft des Wunddistanzgitters, im Sinne einer atraumatischen Wundauflage von der Wundoberfläche abgelöst werden zu können, ohne zu Blutungen, Schmerzen oder Lösungsprozessen von verklebten Oberflächen zu führen.

Die abgewinkelten, an der rauen Oberfläche befindlichen Abschnitte bewirken dabei überdies eine Verminderung des Rückflusses bereits durch das Wunddistanzgitter hindurchgetretener Wundflüssigkeiten, und tragen so dazu bei, die Wunde trocken zu halten.

Der Einsatz des Wunddistanzgitters führt überdies zur Reinhaltung von mikroperforierten Oberflächen des innenliegenden Wundflüssigkeiten absorbierenden Körpers, da grobe Verunreinigungen wie Fibrinbeläge, Verkrustungen oder putride Prozesse nicht direkt nach innen gelangen, sondern im Wundgrund bleiben. Durch diesen Effekt hält das Wunddistanzgitter bei verschmutzten Wunden die Funktion des Wundflüssigkeiten absorbierenden Körpers aufrecht und verlängert seine Applikationsdauer oder ermöglicht die Aufnahme seiner Funktion, da die o. g. Verunreinigungen an den Flächen des Wunddistanzgitters verbleiben, ohne die Löcher zu verlegen.

Die abgewinkelten, an der rauen Oberfläche befindlichen Abschnitte bewirken eine wünschenswerte Verminderung des Rückflusses bereits eingetretener Wundflüssigkeiten.

Die Aufbringung mit der rauen Seite zur Wunde führt dazu, dass das Wunddistanzgitter aktiv bei Bewegung an der Wunde scheuert, die Exsudation und damit das ausspülen von Pathogenen etc fördert und ferner einen erwünschten taktischen Reiz auf die Gewebeneubildung ausüben kann.

Variante b) ist insbesondere dann interessant, wenn die nicht flüssigkeitsabsorbierende Hülle eine Wäscheschutzfunktion aufweist.

Hierbei handelt es sich häufig um ein Blatt aus einem wasserundurchlässigen Material, das häufig mit der Hülle verklebt ist. Besagter Wäscheschutz ist häufig farbig gekennzeichnet, z.B. in Grün, um eine seitenrichtige Auflage der Wundauflage auf die Wunde zu gewährleisten.

Trotz des hohen Aufnahmevermögens für Flüssigkeiten kann in einigen Fällen nicht ausgeschlossen werden, dass aufgenommenes Exsudat aus dem Wundexsudate absorbierenden Körper - ggf. sogar aus dem Wundpflegeartikel - austritt. Dies kann für den Patienten unangenehm sein, da Exsudat mitunter gefärbt ist oder aber unangenehm riechen kann, und unter Umständen kann dies auch für die Umwelt eine Gefährdung darstellen, nämlich dann, wenn das Exsudat mit pathogenen Erregern kontaminiert ist. In diesem Fall beugt die genannte Wäscheschutzfunktion vor.

Ebenso kann aber auch vorgesehen sein, dass die nicht flüssigkeitsabsorbierende Hülle um die flüssigkeitsabsorbierende Hülle herum angeordnet ist, also eine Hülle um die flüssigkeitsabsorbierende Hülle ausbildet.

In einer bevorzugten Ausgestaltung ist dabei vorgesehen, dass die Wundauflage überdies eine den Wundflüssigkeiten absorbierenden Körper wenigstens teilweise umgebende Hülle aufweist, die nicht flüssigkeitsabsorbierend ist, und die
a) als Wunddistanzgittter fungiert, und/oder die
b) eine Wäscheschutzfunktion aufweist.

Dabei können die flüssigkeitsabsorbierende Hülle und die nicht flüssigkeitsabsorbierende Hülle Teil einer Gesamthülle sein. Dabei kann die so gebildete Außenhülle komplett aus dem erwähnten Wunddistanzgitter bestehen, oder die Unterseite (also die der Wunde zugewandte Seite) der Außenhülle fungiert als Wunddistanzgittter, und die Oberseite (also die der Wunde abgewandte Seite) der weist eine Wäscheschutzfunktion auf.

Die flüssigkeitsabsorbierende Hülle kann geschlossen oder offen sein. Als Hülle soll ein flächenhafter, prismatischer, zylindrischer, kugeliger oder torusförmiger Hohlkörper verstanden werden, der aus hochabsorbierendem Material gefertigt ist. Es wird angenommen, dass das hochabsorbierende Material im Sinne der vorliegenden Erfindung ein Material ist, dessen Aufnahmevermögen für Wundexsudat wenigstens das 3-fache des Eigengewichtes beträgt.

Solche hochabsorbierenden Materialien werden auch als "hydroaktive Polymere" bezeichnet, Hierunter sollen im folgenden Polymere verstanden werden, die einerseits eine hohe Wasseraufnahmekapazität aufweisen und andererseits imstande sind, eine atmosphärische Rückfeuchtung zu bewirken. Dies sind unter anderem
- Offenzellige Schäume aus Polyurethan
- Matten, Gelege oder Vliese aus Alginaten
- Matten, Gelege oder Vliese aus Carboxymethylcellulose, insbesondere Natriumcarboxymethylcelluose

Alginate werden aus den Braunalgen gewonnen und zu einem faserigen Vlies verwoben Chemisch handelt es sich um Polysaccharide, und zwar Calcium- und/oder Natriumsalze der Alginsäuren. Alginate können bis zum 20-fachen ihres Eigengewichtes an Flüssigkeit aufnehmen, dabei wird das Wundexsudat in die Hohlräume eingelagert. Die im Alginatgitter enthaltenen Ca²⁺-Ionen werden gegen die Na⁺-Ionen aus dem Exsudat ausgetauscht, bis der Sättigungsgrad an Na-Ionen im Alginat erreicht ist. Dabei kommt es zu einem Aufquellen der Wundauflage und zur Umwandlung der Alginatfaser in einen Gelkörper durch Aufquellen der Fasern.

Carboxymethylcellulose liegt insbesondere in Form von Natriumcarboxymethylcellulose vor und ist unter dem Namen "Hydrofaser" im Handel. In Hygiene- und Wundprodukten werden die Fasern in eine flächeige Matrix überführt. Durch die Aufnahme von Flüssigkeit aus dem Wundexsudat werden die Fasern nach und nach in ein Gelkissen umgewandelt, das die Flüssigkeit hält und nicht wieder freigibt. Dabei sind die Fasern so aufgebaut, dass das Wundexsudat nur in vertikaler Richtung aufgenommen wird. Dies bedeutet dass, solange die Kapazität reicht, das Exsudat nicht über den Wundrand fließt. Auf diese Weise kann eine Wundrandmazeration effektiv verhindert werden.

Besonders bevorzugt ist jedoch vorgesehen, dass es sich bei den hydroaktiven Polymeren um superabsorbierende Polymere handelt.

Superabsorbierende Polymere (SAP) sind Kunststoffe, die in der Lage sind, ein Vielfaches ihres Eigengewichts - bis zum 1000-fachen - an Flüssigkeiten aufzusaugen. Chemisch handelt es sich dabei um ein Copolymer aus Acrylsäure (Propensäure, C₃H₄O₂) und Natriumacrylat (Natriumsalz der Acrylsäure, NaC₃H₃O₂), wobei das Verhältnis der beiden Monomere zueinander variieren kann. Zusätzlich wird ein so genannter Kernvernetzer (Core-Cross-Linker, CXL) der Monomerlösung zugesetzt, der die gebildeten langkettigen Polymermoleküle stellenweise untereinander durch chemische Brücken verbindet (sie "vernetzt"). Durch diese Brücken wird das Polymer wasserunlöslich. Beim Eindringen von Wasser oder wässrigen Salzlösungen in den Polymerpartikel quillt er auf und strafft auf molekularer Ebene dieses Netzwerk, so dass das Wasser ohne Hilfe nicht mehr entweichen kann.

Insbesondere die Superabsorbierenden Polymere (SAP) können in Form einer Schüttung vorliegen, wobei der Begriff "Schüttung" sowohl Granulate und Pulver als auch Materialstücke, wie Schaumstücke, umfassen kann. Die Materialstücke können auch aus bekannter Airlaid-Matte entsprechend geschnitten oder ausgestanzt sein. Die Versuche haben gezeigt, dass die durch Schneiden oder Stanzen entstandenen Materialstücke (Schnipseln) die Absorptionsschnelligkeit um etwa 15% bis 17%, bezogen auf den ganzen Materialabschnitt von demselben Gesamtgewicht vergrößern können.

Der hydroaktive Effekt kann durch Dickungsmittel verbessert werden. Dies können insbesondere Alginsäure (E 400), Agar (E 406), Carrageen (E 407), Johannisbrotkernmehl (E 410), Guarkernmehl (E 412), Traganth (E 413), Gummi arabicum (E 414), Xanthan (E 415), Karaya (E 416), Tarakernmehl (E 417), Gellan (E 418), Pektin (E 440), Zellulose (E 460), Zelluloseether, Carboxymethylcellulose (E 466), Hydroxypropylcellulose (E 463), Hydroxypropylmethylcellulose (E 464), Methylcellulose (E 461), Methylethylcellulose (E 465), modifizierte Stärke (Gruppe: E 1404, E 1410, E 1412, E 1413, E 1414, E 1420, E 1422, E 1440, E 1442, E 1450, E 1451).

Der absorbierende Körper kann aus wenigstens einer cellulosehaltigen Matte, beispielsweise von Typ Airlaid, bestehen, die vorzugsweise lose innerhalb der Hülle liegt. Vorzugsweise ist die Matte flächenmäßig kleiner als die Hülle, falls die letztere flächenhaft ist. Die Matte kann aus Schaumstoff, beispielweise aus Polyurethanschaum bestehen, der insbesondere offenzellig ist.

Besonders bevorzugt ist vorgesehen, dass die absorbierenden Materialien, insbesondere die superabsorbierenden Polymere, in Form eines Granulats, eines Pulvers, einer Schüttung, eines Presslings, eines Schaums, in Form von Fasern, eines Fasergewirkes, -geleges oder - vlieses und/oder einer Faserwatte vorliegen.

Die Granulat- oder Pulverform hat sich sehr bewährt und ist daher besonders bevorzugt, da sie sich gut in ein Vlies aus einem Fasermaterial einbringen lässt. Hier ist insbesondere an eine sogenannte Airlaidmatte gedacht.

Eine Schüttung kann insbesondere aus in kleine Stücke zerschnittenem Schaum., insbesondere PU-Schaum, bestehen.

Auch dieser kann geschnitten oder gestanzt sein, sodass sogenannte Schnipsel vorliegen, die ähnliche Vorteile aufweisen wie oben bereits geschildert.

Ebenso ist jedoch insbesondere bei Superabsorbierenden Polymeren die Faserform besonders bevorzugt, da es sich hierbei um ein sowohl im trockenen als auch im gequollenen Zustand sehr weiches Produkt handelt, das modellierbar und nicht steif ist und das im Gegensatz zu den in Granulat- oder Pulverform vorliegenden superabsorbierenden Polymeren überdies eine geringe Abrasivität aufweist. Dies gilt sowohl für Fasern als solches als auch für Fasergewirke, -gelege oder -vliese und/oder Faserwatte.

Die besagten Eigenschaften machen superabsorbierende Polymere in Faserform insbesondere für erfindungsgemäße Produkte geeignet, die als Analtamponade, Wundfüller und Kavitätentamponade ausgestaltet sind. Gerade bei diesen Anwendungsbereichen kommt es besonders auf hohe Weichheit an.

Überdies können die superabsorbierenden Polymere in Faserform einen Dochteffekt aufweisen bzw. unterstützen, der insbesondere die Exsudatableitung aus besagten Wunden unterstützt.

Weiterhin, so hat die Anmelderin beobachtet, weisen superabsorbierende Polymere in Faserform ein schnelleres Ansprechverhalten gegenüber Flüssigkeiten auf als superabsorbierende Polymere in Granulat- oder Pulverform.

Der absorbierende Körper weist bevorzugt ein flächenspezifisches Gewicht von 100 g/m² - 600 g/m² auf, wobei der darin verteilte Anteil der Hydroaktiven, insbesondere superabsorbierenden Polymeren im Bereich von 25 - 100 Gew.-% liegt.

Die Hülle wird aus polymerem, synthetischem Weichschaumstoff, insbesondere aus Polyurethanschaum hergestellt. Hierbei ist es denkbar, die Hülle aus Integralschaum herzustellen, indem die Dichte des Schaumstoffs im Querschnitt der Matte sich ändert. Das Material für die Hülle kann in einer Sandwichanordnung vorliegen. Hierbei wird auf die an sich bekannte Airlaid-Matte mit perforierten Deckschichten verwiesen, wobei die Matte wenigstens eine dehnbare, vorzugsweise plissierte oder gekreppte Deckschicht aufweisen kann.

Eine Sandwichanordnung des Hüllenmaterials kann wenigstens eine Schaumschicht und wenigstens eine folienartige, perforierte bzw. maschenartige Deckschicht aufweisen, die ebenfalls gekreppt oder plissiert sein kann. Die Deckschicht kann auch in Form eines Textilmaterials, wie Gewebe oder Vlies, vorliegen.

Das Hüllenmaterial kann mit wenigstens einer superabsorbierenden Substanz versehen sein, die ebenfalls in Pulver-, Granulat- oder Faserform vorliegen kann.

Die flächige bzw. punktuelle Verbindung zwischen der Deckschicht und dem absorbierenden Körper ist bevorzugt durch Klebungen, Verschweissungen, Nähte, Steppnähte, Bonding Points, Prägungen oder durch thermomechanische Bindungen bewerkstelligt.

Klebungen können dabei insbesondere flächig oder punktuell mit physiologisch akzeptierbaren Klebemitteln, wie z.B,. Stärkekleber, Eiweißkleber, Acrylatkleber und dergleichen ausgeführt sein.

Steppnähte können beispielsweise auch mit elastischem Garn ausgeführt werden. Auf diese Weise wird eine Expansion des absorbierenden Körper ermöglicht, wenn dieser Flüssigkeit aufnimmt.

Bei besagten Bonding Points handelt es sich um Presspunkte, die mithilfe einer Presse auf den Wundpflegeartikel aufgebracht wurden, und die für eine thermische und/oder physikalische Verbindung der verschiedenen Schichten des Wundpflegeartikels sorgen. Die Bonding points liegen in der Regel in einem regelmäßigen Muster vor.

Prägungen sind Verbindungen, die ausschließlich durch Ausübung von Druck zustande kommen.

Unter dem Begriff "thermomechanische Bindungen" werden im Folgenden Verbindungen verstanden, die unter Auswirkung von Druck und Wärme zustande kommen. Dabei kann sich durch die Steppnähte, Prägungen oder thermomechanische Bindungen insbesondere ein wabenförmiges, rautenförmiges oder kariertes Muster ergeben.

Bei einem sehr hohen Anteil superabsorbierenden Polymeren kann eine eventuell vorgesehene Decksicht aus Zellulose nicht über Bonding Points mit der Lage enthaltend superabsorbierende Polymere verbunden werden. In diesen Fällen ist es erforderlich, beide Lagen über eine adhäsive Bindung miteinander zu verbinden.

### Weitere Ausgestaltungen

Bevorzugt ist in diesem Zusammenhang außerdem vorgesehen, dass der Wundpflegeartikel außerdem mindestens einen nutritiven, mindestens einen desinfizierenden bzw. dekontaminierenden und/oder mindestens einen Proteasen hemmend wirkenden Wirkstoff und/oder Wirkstoffkomplex aufweist.

Bei dem desinfizierend wirkenden Wirkstoff und/oder Wirkstoffkomplex kann es sich z.B. um eine Zusammensetzung aus mindestens einem Vitamin oder Vitaminderivat, einem Metallion sowie einem Detergenz handeln. Ebenso kann es sich dabei um einen BLIS (bacteriocin like inhibitory substance) oder um beschichtete magnetische Partikel handeln, wie auch um Silberdonatoren, handeln, wie z.B. Silberionen.

Bei dem nutritiv wirkenden Wirkstoff und/oder Wirkstoffkomplex kann es sich um eine Zusammensetzung enthaltend mindestens die Bestandteile eines enteralen und/oder parenteralen Diätetikums handeln. Ebenso kann es sich dabei um mindestens ein Wirkelement ausgewählt aus der Gruppe enthaltend Insulin, rekombinantes Insulin, Proinsulin, einen insulinähnlichen Wachstumsfaktor (Insulin-like growth factor, IGF), ein Insulinmimetikum und/oder einen diabetikerspezifischen, nicht glucose- bzw. saccharosebasierenden Energieträger handeln.

Bei dem Proteasen hemmend wirkenden Wirkstoff und/oder Wirkstoff komplex kann es sich um mindestens ein Wirkelement ausgewählt aus der Gruppe enthaltend Proteasehemmer, superabsorbierende Polymere, Chelatoren für zweiwertige Kationen, Kollagen, beschichtete magnetische Partikel, Säuren, Puffer, nicht pathogene säureproduzierende Mikroorganismen, Probiotika und/oder Symbiotika handeln.

Überdies kann die Zusammensetzung auch analgetische, d.h. schmerzstillende Wirkstoffe enthalten. Hier kommen prinzipiell alle jene Wirkstoffe in Frage, die in Hauptgruppe 05 der sog. "Roten Liste" aufgeführt sind. Besonders bevorzugt sind dabei insbesondere entzündungshemmende Wirkstoffe wie z.B. sogenannte Cox-Hemmer oder NSAID (non steroidal anti-inflammatroy drugs), so z.B. Propionsäurederivate wie Naproxen, Ibuprofen, Ketoprofen, Fenoprofen, Flurbiprofen, Dexibuprofen oder Tiaprofensäure, Essigsäurederivate wie z.B. Diclofenac, Alclofenac, Fenclofenac, Etodolac, Aceclofenac, Sulindac oder Indometacin, Pyrrolessigsäuren wie Ketorolac oder Tolmetin, N-Phenylessigsäuren wie Mefenaminsäure oder Flufenaminsäure, Salicylate wie Acetylsalicylsäure, Salicylsäure oder Diffunisal, Pyrazolonderivate wie Phenylbutazon, Oxicamderivate wie Piroxicam, Tenooxicam, Meloxicam oder Lornoxicam, Enolsäurederivate wie Aminopyren oder Antipyren, Phenole wie Acetaminophen und dergleichen. Hinzu kommen COX-2-Inhibitoren wie beispielsweise Rofecoxib, Lumiracoxib oder Celecoxib.

Überdies kann es sich bei den schmerzstillenden Wirkstoffen auch um nicht entzündungshemmende Wirkstoffe handeln, so z.B. um Opiate, lokale Anaesthetika wie Lidocain, Mepivacain, Prilocain, Procain, Syntocain, Tetracain, Gingicain, Articain, Bupivacain, Butanilicain, Chloroprocain, oder z.B. Polidocanol.

Weiterhin kann die Zusammensetzung auch entzündungshemmende Wirkstoffe aufweisen, die ggf. sekundär einen analgetischen Effekt aufweisen, so z.B. - neben den oben genannten, z.T. ebenfalls entzündungshemmend wirkenden Analgetika - Hormone, insbesondere Cortison und Cortikoide, insbesondere Glucokortikoide (z. B. Cortison, Cloprednol, Prednison, Prednisolon, Methylprednisolon, Deflazacort, Fluocortolon, Triamcinolon, Dexamethason, Betamethason) und Mineralokortikoide (z.B. Aldosteron, Desoxycorticosteron, Fludrocortison).

Weitere Zusammenhänge und Hintergründe zu den nutritiven, einen desinfizierenden bzw. dekontaminierenden und/oder Proteasen hemmend wirkenden Wirkstoffen und/oder Wirkstoff komplexen sind in der DE 102007030931 der Anmelderin der vorliegenden Anmeldung beschrieben. In der DE 102007030931 sind auch weitere nutritive, desinfizierende bzw. dekontaminierende und/oder Proteasen hemmend wirkende Wirkstoffen und/oder Wirkstoffkomplexe beschrieben.

Weiterhin kann der erfindungsgemäße Wundpflegeartikel auch in ein Wundversorgungssystem zur Wunddrainage unter Einsatz von Unterdruck eingebracht sein. Solche Systeme sind z.B. in den Schriften DE202004017052, WO2006048246 und DE202004018245 der Anmelderin der vorliegenden Erfindung offenbart.
Aus erstgenannter ist eine Vorrichtung zur Wundbehandlung unter Einsatz von Unterdruck bekannt, aufweisend ein gasdichtes Wundabdeckungselement, das im am Körper des Patienten angelegten Zustand einen zwischen der jeweiligen Wunde und dem Wundabdeckungselement verbleibenden Raum bildet, und wenigstens eine Anschlussstelle, die mit dem Raum in Kontakt steht und über welche die im Raumbefindliche Luft evakuiert werden kann, wobei das Wundabdeckungselement von wenigstens einem flächenhaften, die Wundsekrete aufnehmenden Wundpflegeartikel unterlegt ist, dessen Volumen im Laufe des Absorptionsprozesses zunimmt, so dass die absorbierten Wundsekrete innerhalb des Wundpflegeartikelsund damit unterhalb des Wundabdeckungselementes bis zur Entfernung des Wundpflegeartikels aus dem Körper des Patienten verbleiben, der Wundpflegeartikel wenigstens eine Lage eines mit Superabsorbentien angereicherten Textilabschnittes ist, die mit einer flüssigkeitsdurchlässigen Hülle umgeben ist, und die Lage in Draufsicht auf ihre Flachseite eine Fläche hat, die 3 % bis 90 % kleiner als die der Hülle ist, damit sich der Wundpflegeartikel in der Nähe seiner gesamten Füllungskapazität im Querschnitt einer Kreisform annähern kann.

Aus zweitgenannter ist ein Mehrkomponentenverband zur Wundbehandlung des menschlichen oder tierischen Körpers unter Einsatz von Unterdruck bekannt, aufweisend: ein Wundabdeckungselement zur Anbringung an Haut- und Schleimhautoberfläche, wenigstens eine Anschlussstelle, die mit dem Wundraum in Kontakt steht und über welche die im Wundraumbefindlichen Stoffe evakuiert werden können, wobei dieser superabsorbierende Polymere auf weist, wobei die absorbierten Wundsekrete an Polymere gebunden im Wundraum bis zur Entfernung aus dem Wundraum verbleiben, wobei die Polymere durch ihre Bindungskapazität wechselseitige Synergien mit den subatmosphärischen Drücken unterstützen.

Aus letztgenannter ist eine Drainagevorrichtung zur Wundbehandlung unter Einsatz von Unterdruck bekannt, aufweisend ein gasdichtes, aus folienartigem Material bestehendes Wundabdeckungselement, das im am Körper des Patienten angelegten Zustand an der Hautoberfläche um den Wundbereich herum adhäsiv befestigt ist und einen zwischen der jeweiligen Wunde und dem Wundabdeckungselement verbleibenden, abgedichteten Raum bildet, wenigstens einen Drainageschlauch, der in den Raumeinsetzbar ist, über den die im Raumbefindlichen Stoffe evakuiert werden können, und wenigstens einen innerhalb des Raumes angeordneten, die Wundsekrete aufsaugenden Wundpflegeartikel, der wenigstens eine Lage eines mit Superabsorbentien angereicherten Textilabschnittes aufweist, die mit einer flüssigkeitsdurchlässigen Hülle umgeben ist, wobei die absorbierten Wundsekrete innerhalb des Wundpflegeartikel und damit unterhalb des Wundabdeckungselementes bis zur Entfernung des Wundpflegeartikel aus dem Körper des Patienten verbleiben, und wobei das Wundabdeckungselement eine gasdicht verschliessbare Behandlungsöffnung aufweist, durch die der Wundpflegeartikel in den Raum einlegbar und aus dem Raum entnehmbar ist.

Der Erfindungsgemäße Wundpflegartikel kann überdies eine an anatomische Gegebenheiten angepasste Form aufweisen. Hierzu kann er z.B. in Form einer Manschette ausgebildet sein; die über den einen Arm oder ein Bein oder ein Gelenk gestülpt werden kann, oder in Form eines an die Ferse, das Ellenbogengelenk oder dergleichen angepaßten Verbandes.

Der erfindungsgemäße Wundpflegartikel kann außerdem so ausgebildet sein, dass er sich zur Umlage um eine chirurgisch angelegte Leitung eignet. Hierzu kann der Wundpflegeartikel z.B. wenigstens einen Schlitz aufweisen, der es ermöglicht, den Verband am Körper eines Patienten um eine Leitung (z.B. eine Drainageleitung oder einen Kathether) umzulegen, wobei dem Wundpflegeartikel ein zweiter, ebenfalls flächenhafter Wundpflegeartikel zugeordnet ist, der von dem ersten Wundpflegeartikel in einem Abstand liegt, wobei der Abstand durch einen Verbindungsstreifen oder -steg überbrückt ist. Ein solcher Wundpflegartikel ist z.B. aus der DE202006005966 der Anmelderin der vorliegenden Erfindung bekannt.

Ebenso ist in diesem Zusammenhang bevorzugt vorgesehen, dass der Wundpflegeartikel mindestens ein Agenz aufweist, das die Blutung oder die Blutungsneigung einschränken kann. Bei besagtem Agenz kann es sich um mindestens einen chemisch und/oder physiologisch wirkenden Wirkstoff bzw. Wirkstoffkomplex oder um mindestens ein physikalisch wirkendes Wirkelement handeln. Ein solcher Wundpflegeartikel ist z.B. aus einer parallel eingereichten Anmeldung der Anmelderin der vorliegenden Anmeldung bekannt.

Hierzu kann der Wundpflegeartikel beispielsweise
- als im wesentlichen flacher Materialabschnitt aufweisend Absorptionsmaterial, der aus einem aufsaugenden Vlies mit darin verteilten superabsorbierenden Polymeren sowie mindestens einem chemisch und/oder physiologisch wirkende Wirkstoff bzw. Wirkstoff komplex ausgebildet sein,
- als oder in Kombination mit einem Druck- oder Kompressionsverband,
- als eine Kombination aus einer primären, nicht oder nur unwesentlich absorbierenden Wundauflage, die mindestens einen chemisch und/oder physiologisch wirkenden Wirkstoff bzw. Wirkstoff komplex aufweist, und einer peripher von dieser primären Wundauflage angeordneten sekundären Wundauflage, die superabsorbierende Polymere enthält, wobei ggf. zwischen beiden eine Diffusionsbarriere angeordnet ist,
- in Form eines Verbandpäckchens, aufweisend eine primäre Wundauflage mit mindestens einem chemisch und/oder physiologisch wirkenden Wirkstoff bzw. Wirkstoffkomplex sowie einem an der Wundauflage angeordneten Wickelabschnitt, der zumindest abschnittsweise superabsorbierende Polymere aufweist, und/oder
- als Materialabschnitt mit einer Längserstreckung aufweisend Absorptionsmaterial, wobei der Materialabschnitt elastisch verformbare Eigenschaften aufweist, und wobei der Materialabschnitt superabsorbierende Polymere sowie ggf. mindestens einen chemisch und/oder physiologisch wirkende Wirkstoff bzw. Wirkstoffkomplex aufweist

Bevorzugt handelt es sich bei dem chemisch und/oder physiologisch wirkenden Wirkstoff bzw. Wirkstoff komplex um mindestens einen Stoff bzw. eine Zusammensetzung, die Blutstillende Eigenschaften aufweist. Diese Stoffe sind unter dem Oberbegriff "Hämostatikä und/oder "Antihämorrhagika" bekannt.

Bei dem physikalisch wirkenden Wirkelement handelt es sich z.B. um eine Abbindung, ein Druckpolster, einen Druckverband oder einen Kompressionsverband.

In weiteren Ausgestaltungen des erfindungsgemäßen Wundpflegeartikels ist vorgesehen, dass
- der innerhalb der Hülle angeordnete absorbierende Körper (1; 11; 21) ein Airlaid mit hydroaktiven, bevorzugt superabsorbierenden Polymeren in Pulver- oder Granulatform aufweist, während die Hülle hydroaktive, bevorzugt superabsorbierende Polymere in Faserform aufweist; sowohl die Hülle als auch der innerhalb der Hülle angeordnete absorbierende Körper (1; 11; 21) Airlaid-Material mit darin angeordneten hydroaktiven bevorzugt superabsorbierenden Polymeren aufweisen;
- die Hülle einen Schaumstoff und der innerhalb der Hülle angeordnete absorbierende Körper (1; 11; 21) Airlaid-Material mit darin angeordneten hydroaktiven, bevorzugt superabsorbierenden Polymeren aufweist;
- sowohl die Hülle als auch der innerhalb der Hülle angeordnete absorbierende Körper (1, 11; 21) Airlaid-Material mit darin angeordneten hydroaktiven, bevorzugt superabsorbierenden Polymeren aufweisen; und/oder
- der innerhalb der Hülle angeordnete absorbierende Körper (1; 11; 21) ein Airlaid mit superabsorbierenden Polymeren in Pulver- oder Granulatform aufweist, während die Hülle superabsorbierende Polymere in Faserform aufweist.

Besonders bevorzugt ist vorgesehen, dass wenigstens ein Bestandteil des Wundpflegeartikels zu wenigstens einem anderen Bestandteil Wundpflegeartikels relativ beweglich ist.

In dieser Ausgestaltung kann z.B. vorgesehen sein, dass absorbierende Körper lose im Inneren der Hülle angeordnet ist.

In einer weiteren Ausgestaltung ist vorgesehen, dass die Hülle in Form einer mindestens einseitig offenen, ggf. verschließbaren Tasche ausgebildet ist. In besagter Hülle kann dann im Sinne der Erfindung ein absorbierender Körper angeordnet werden, der nach der jeweiligen Wundsituation aufgrund seiner Absorptionseigenschaften ausgewählt werden kann.

Weiterhin ist in einer ebenfalls bevorzugten Ausgestaltung vorgesehen, dass die Hülle in Form einer einseitig offenen Tasche ausgebildet ist, zumindest abschnittsweise eine luftdichte Schicht sowie mindestens eine Einrichtung zum Anschluß einer Absaugeinrichtung aufweist.

In dieser Ausgestaltung kann der Wundpflegeartikel für die Unterdrucktherapie einer Hand, eines Fußes eines Arms, eines Beins oder dergleichen verwendet werden. Eine solche Unterdrucktherapie ist insbesondere bei Verbrennungen angezeigt, die zu starker Exsudation neigen.

Hierfür kann die Hülle des Wundpflegeartikels im Bereich ihrer Öffnung eine Manschette aufweisen, durch welche z.B. die Hand hindurchgefühlt und mit welcher dann ein luftdichter Anschluß hergestellt werden kann. Die Einrichtung zum Anschluß einer Absaugeinrichtung kann z.B. aus einem durch die Hülle hindurchgeführten Stutzen bestehen, an welchem ein Absaugschlauch angeordnet werden kann. Ebenso kann die Einrichtung zum Anschluß einer

Absaugeinrichtung auch in besagte Manschette integriert sein, dergestalt, dass hier der ein Absaugschlauch hindurchgefühlt werden kann.

### Definitionen

Der Begriff' Wundpflegeartikel" soll im Folgenden insbesondere eine Wundauflage, bevorzugt eine flächige Wundauflage oder ein Wundpflegetuch bezeichnen. Besagte Wundauflage kann dabei sowohl absorbierend als auch nicht oder nur unwesentlich absorbierend ausgestaltet sein. Insbesondere kann der Begriff" Wundpflegeartikel" auch als ein Ensemble verschiedener Produkte verstanden werden, die in einer gegebenen Anordnung auf der zu behandelnden Wunde angeordnet werden. Dieses Ensemble kann eine physikalische Einheit bilden, indem die verschiedenen Produkte in einer gemeinsamen Hülle zusammengefasst oder - ggf. ohne Hülle - adhäsiv miteinander verbunden sind. Das Ensemble kann jedoch auch in Form eines Kits vorliegen, bei dem die verschiedenen Produkte mithilfe einer Wickel in der gegebenen Anordnung auf der zu behandelnden Wunde angeordnet sind.

Unter dem Begriff "chronische Wunden" sollen Wunden verstanden werden, die nicht primär auf traumatische Einwirkungen zurückgehen. Zwar können traumatische Einwirkungen der ursprüngliche Auslöser einer solchen Wunde gewesen sein, aber die chronische Wunde zeichnet sich vor allem durch eine verzögerte Wundheilung auf. Chronische Wunden weisen häufig - wenn überhaupt - nur leichte Blutungen auf, dafür oftmals eine starke Exsudation.

### Zeichnungen

Die vorliegende Erfindung, wie in den Ansprüchen definiert, wird durch die im Folgenden gezeigten und diskutierten Figuren und Beispiele genauer erläutert. Dabei ist zu beachten, dass die Figuren und Beispiele nur beschreibenden Charakter haben und nicht dazu gedacht sind, die Erfindung in irgendeiner Form einzuschränken. Die Figuren zeigen:
- Fig. 1: einen flächenhaften Wundpflegeartikel in einer perspektivischen Ansicht;
- Fig. 2: einen ebenfalls flächenhaften Wundpflegeartikel, beidseitig offen, in einer schematischen Draufsicht auf seine Flachseite;
- Fig. 3: ein schaumartiges Hüllenmaterial mit einer aufkaschierten Deckschicht, in einem schematischen Schnitt;
- Fig. 4: das schaumartige Hüllenmaterial mit beidseitig aufkaschierten Deckschichten, in einem schematischen Schnitt;
- Fig. 5: eine andere Ausführungsform des flächenhaften Wundpflegeartikels, mit einer umgekrempelten Hülle, in einem schematischen Schnitt;
- Fig. 6: einen flächenhaften Wundpflegeartikel gemäß Fig. l, jedoch mit einer faserigen Füllung, in einer perspektivischen Ansicht;
- Fig. 7: einen für Wundtaschen bestimmten, etwa zylindrisch ausgebildeten Wundpflegeartikel, in einer perspektivischen Ansicht;
- Fig. 8: einen weiteren Wundpflegeartikel für tiefe Wunden, wie Kavitäten, bestimmten Wundpflegeartikel, in einer perspektivischen Ansicht;
- Fig. 9: einen torusförmigen Wundpflegeartikel, in einer perspektivischen Ansicht;
- Fig. 10: einen Schnitt A-A gemäß Fig. 9;
- Fig. 11: eine Hülle mit Verschlußlasche, in einer schematischen perspektivischen Ansicht,
- Fig. 12: eine Schlitzkompresse in Draufsicht auf ihre Flachseite,
- Figs. 13 - 15: zeigen weiterer Ausgestaltungen eines erfindungsgemäßen Wundpflegeartikels

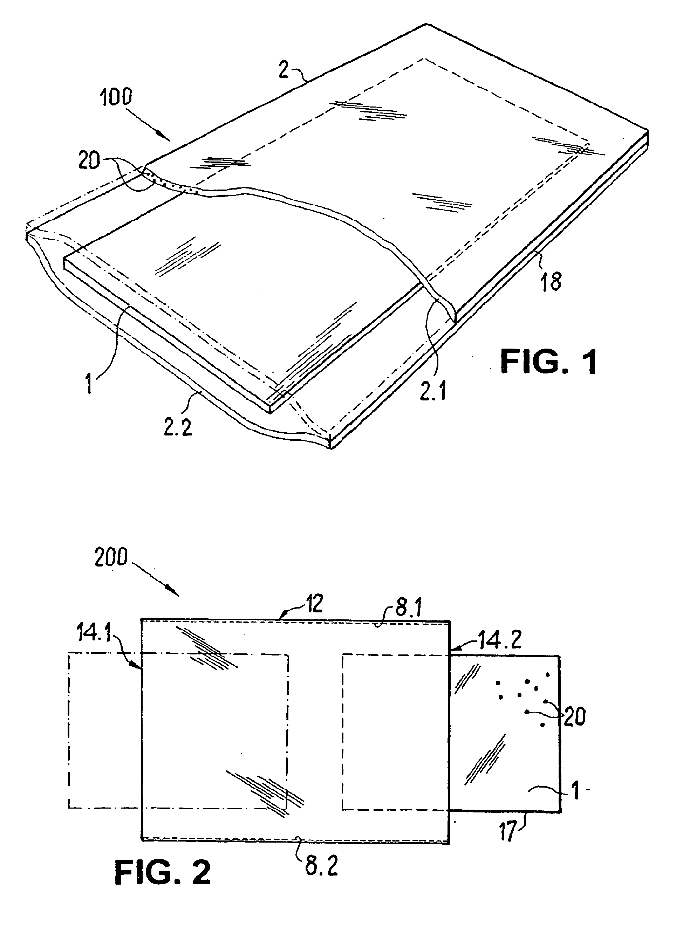

In Fig. 1 ist ein flächenhafter, rechteckiger Wundpflegeartikel 100 dargestellt, bestehend aus einem das Wundexsudat absorbierenden Körper 1 in Form einer cellulosehaltigen Matte und aus einer die Matte umgebenden Hülle 2. Mit den Bezugszahlen 2.1 und 2.2 sind Hüllenwände bezeichnet. Sowohl die Hülle 2 als auch der in der Hülle 2 befindliche Körper 1 bestehen im vorliegenden Fall aus einer an sich bekannter, mit einer pulverförmigen superabsorbierenden Substanz 20 versehenen Airlaid-Matte. Die Hüllenwände 2.1, 2.2 sind über eine umlaufende Naht 18 miteinander verbunden. Der beschriebene Wundpflegeartikel 100 weist insgesamt eine Dicke von 4mm bis 5 mm auf.

Die Fig. 2 zeigt einen dem oben beschriebenen sehr ähnlichen Wundpflegeartikel 200, bei dem seine Hülle 12 nur zwei sich gegenüber liegende Nähte 8.1, 8.2 aufweist, d. h. sie verfugt über zwei offene Seiten 14.1,14.2 zur Aufnahme des mattenartigen Körpers 1. Da die Matte mit pulverförmiger superabsorbierenden Substanz 20 versehen ist, ist es notwendig, die Matte an seiner Peripherie 17 zu versiegeln.

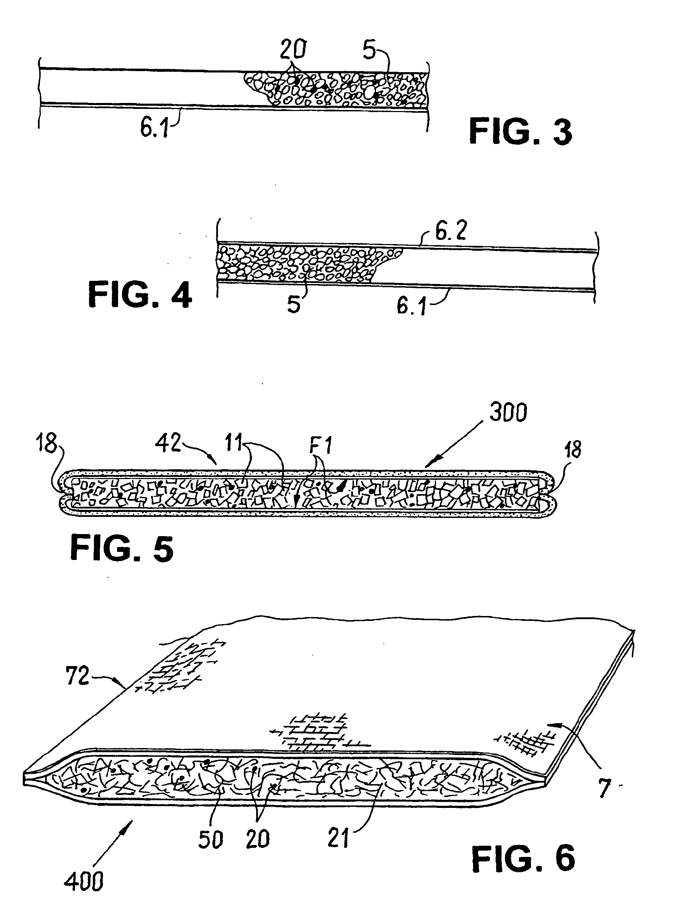

Gemäß der Fig. 3 besteht das Hüllenmaterial aus einer weicher Schaumschicht 5 aus Polyurethan und einer auf die Schaumschicht aufkaschierten Deckschicht 6.1 aus hauchdünner perforierter Polyesterfolie. Die Schaumschicht 5 enthält ebenfalls eine Menge an pulverförmiger superabsorbierenden Substanz 20.

Die Fig. 4 zeigt einen sandwichartigen Aufbau des Hüllenmaterials, mit dem Unterschied, dass die Schaumschicht 5 beidseitig mit Deckschichten 6.1 und 6.2 belegt ist. Die Deckschichten 6.1 und 6.2 sind mit der Schaumschicht 5 ganzflächig fest verbunden.

Ein ebenfalls flächenhafter Wundpflegeartikel 300 ist der Fig. 5 zu entnehmen. Hierbei handelt es sich um eine Hülle 42, die durch Umkrempeln der in der Fig. 2 gezeigten Hülle 12 entstanden ist. Allerdings ist für die Hülle 42 das bei der Fig. 3 beschriebene, schaumartige Hüllenmaterial mit Deckschicht ausgewählt worden. Selbstverständlich ist wenigstens eine der Seiten (vgl. Fig. 2) offen. Bei der fertigen Hülle 42 bildet die Deckschicht eine Innenfläche F1 der Hülle. In der Hülle 42 ist ein absorbierender Körper 11 in Form einer Schüttung aus kleinen würfelförmigen Materialstücken von einer Größe 1,5 mm bis 4,0 mm, die aus der Airlaid-Matte bzw. aus einem Abfallprodukt bei der Herstellung der Matte geschnitten sind. Eine solche Ausführungsform erfordert die Fertigung einer abschließenden Naht.

Die Fig. 6 zeigt einen weiteren Wundpflegeartikel (Bezugszahl 400), bestehend aus einer flächenhaften Hülle 72 und einem aus mehreren Carboxymethylcellulose-Fasern 50 gebildeten absorbierenden Körper 21. Die Carboxymethylcellulose-Fasern 50 sind mit pulverförmiger superabsorbierenden Substanz 20 vermischt, die an den Fasern haftet. Bei einem anderen, nicht dargestellten Ausführungsbeispiel ist der faserige absorbierende Körper ausschließlich aus einer superabsorbierenden Substanz gebildet.

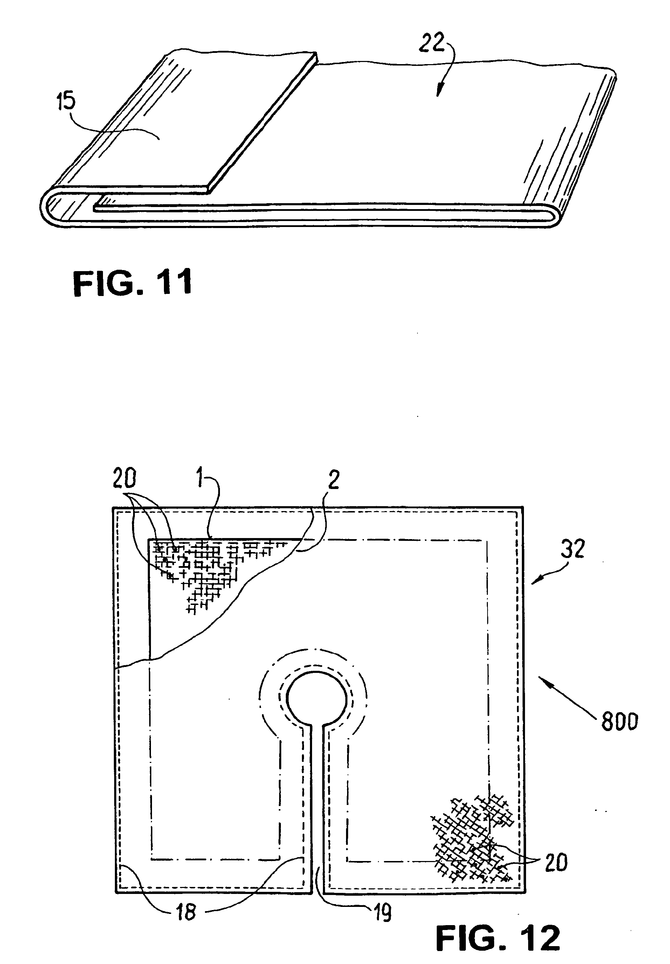

Eine in der Fig. 11 gezeigte Hülle 22 ist mit einer Verschlußlasche 15 versehen, die es ermöglicht, nach Bedarf einen ausgewählten absorbierenden Körper, wie eine Schüttung aus würfelförmigen Alginatstücken oder eine Matte, im Inneren der Hülle zu unterbringen.

Wird an dem in Fig. 1 gezeigten rechteckigen Wundpflegeartikel 100 ein Schlitz 19 eingebracht, so ergibt sich ein Wundpflegeartikel 800 in Form einer neuartigen chlitzkompresse von erhöhter Aufsaugkapazität. Die umlaufende Naht 18 geht in den Schlitzbereich über.

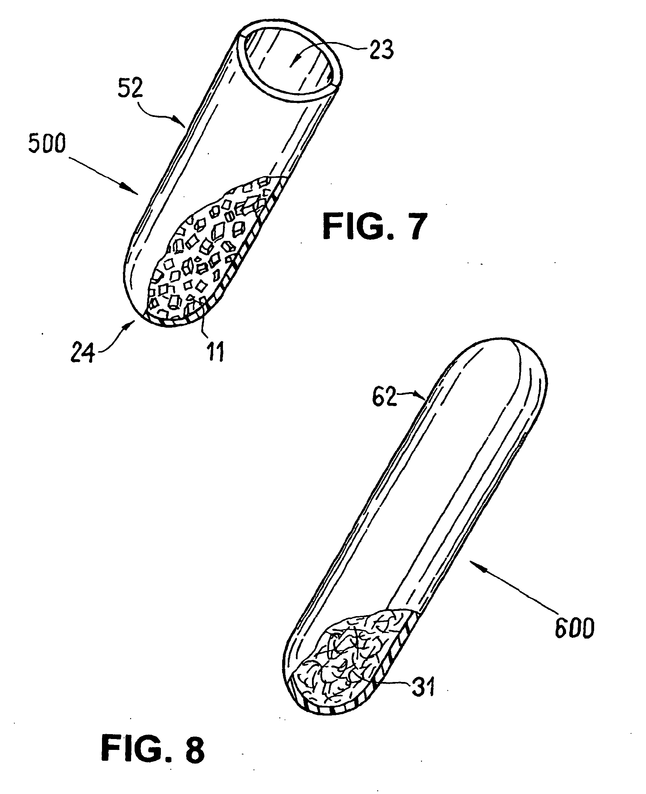

In Fig. 7 ist ein für die tiefen Wunden und Kavitäten bestimmter Wundpflegeartikel 500 dargestellt, bestehend aus einer teilzylindrischen Hülle 52 und dem bereits beschriebenen absorbierenden Körper 11 in Form einer Schüttung aus kleinen würfelförmigen, mit pulverförmiger superabsorbierenden Substanz 20 durchsetzten Materialstücken von einer Größe 2,0 mm bis 4,0 mm. Die Hülle 52 besteht aus 3mm-dickem Weichschaumstoff aus Polyurethan. Die Materialstücke sind durch maschinelles Schneiden einer Airlaid-Matte einer Dicke von 2 mm entstanden. Wie die Figur zeigt, ist die Hülle 52 an einem ihrer Ende 23 offen und an ihrem zweiten, gegenüber liegenden Ende 24 abgerundet und geschlossen.

Ein in Fig. 8 gezeigter Wundpflegeartikel 600 weist eine geometrisch sehr ähnlich ausgebildete, jedoch geschlossene Hülle 62 mit zwei abgerundeten Enden auf. Innerhalb der Hülle 62 ist ein absorbierender Körper 31 als faserige Füllung untergebracht. Die Füllung liegt in Form eines lockeren, luftigen Gebildes vor. Damit ist der wurstförmige Wundpflegeartikel 600 insbesondere gut für die Wundheilung im Analbereich geeignet.

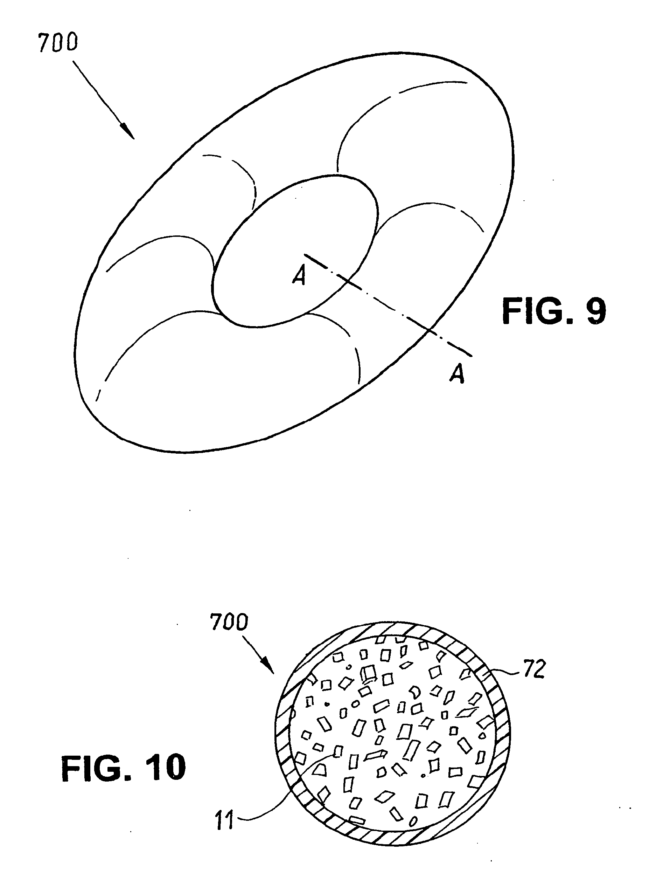

Schließlich zeigen die Figuren 9 und 10 einen torusförmigen Wundpflegeartikel 700, bei dem die aus Polyurethanschaum gefertigte Hülle 72, wie aus der Fig. 10 ersichtlich, in ihrem Querschnitt kreisförmig ist. Das Innenleben (absorbierender Körper 11) besteht aus denselben, bei der Fig. 7 beschriebenen würfelförmigen Materialstücken. Es ist nicht ausgeschlossen, an dem Wundpflegeartikel 700 einen radialen Schnitt (nicht dargestellt) einzuarbeiten, um eine torusförmige Schlitzkompresse herstellen zu können.

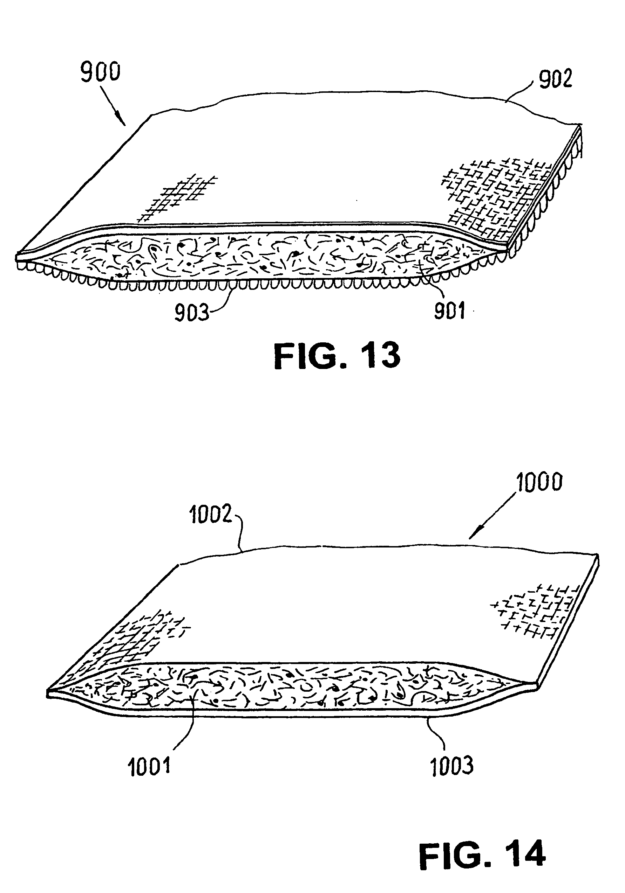

Fig. 13 zeigt einen Wundpflegeartikel 900 mit einem Wundexsudate absorbierenden Körper 901 und mit einer zusammengesetzten Gesamthülle, wobei die flüssigkeitsabsorbierende Hülle als Oberseite 902 (also der Wunde abgewandte Seite) der Gesamthülle fungiert, und die nicht flüssigkeitsabsorbierende Hülle als Unterseite 903 (also der Wunde zugewandte Seite) der Gesamthülle fungieren. Bei der nicht flüssigkeitsabsorbierenden Hülle 903 handelt es sich um ein Wunddistanzgittter.

Fig. 14 zeigt einen Wundpflegeartikel 1000 mit einem Wundexsudate absorbierenden Körper 1001 und mit einer zusammengesetzten Gesamthülle, wobei die nicht flüssigkeitsabsorbierende Hülle als Oberseite 1002 (also der Wunde abgewandte Seite) der Gesamthülle fungiert, und die flüssigkeitsabsorbierende Hülle als Unterseite 1003 (also der Wunde zugewandte Seite) der Gesamthülle fungiert. Die nicht flüssigkeitsabsorbierende Hülle 1002 weist eine Wäscheschutzfunktion auf.

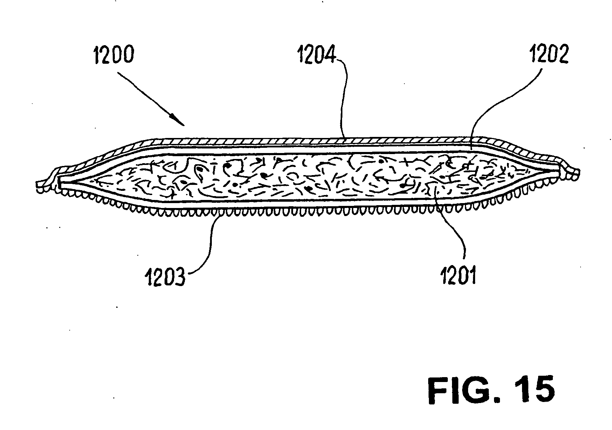

Fig. 15 zeigt einen Wundpflegeartikel 1200 mit einem Wundexsudate absorbierenden Körper 1201 und mit einer flüssigkeitsabsorbierenden Hülle 1202, die von einer zusammengesetzten Aussenhülle umgeben ist, deren Unterseite 1203 (also die der Wunde zugewandte Seite) als Wunddistanzgittter fungiert, und deren Oberseite 1204 (also die der Wunde abgewandte Seite) eine Wäscheschutzfunktion aufweist.

## Patentansprüche

1. Wundpflegeartikel (100; 200; 300; 400; 500), aufweisend wenigstens einem Wundflüssigkeiten absorbierenden Körper (1; 11; 21) und wenigstens eine den Körper (1; 11; 21) wenigstens teilweise umgebenden Hülle (2; 12; 22),
**dadurch gekennzeichnet, dass**
die Hülle (2; 12; 22) selbst flüssigkeitsabsorbierend ist und
die Hülle Weichschaumstoff, vorzugsweise Polyurethanschaum, aufweist,
wobei der absorbierende Körper lose im Inneren der Hülle angeordnet ist.

2. Wundpflegeartikel gemäß Anspruch 1, **dadurch gekennzeichnet, dass** der Wundpflegeartikel im unbenutzten Zustand flächenhaft oder etwa zylindrisch ist.

3. Wundpflegeartikel gemäß einem der vorherigen Ansprüche, **dadurch gekennzeichnet, dass** die Wundauflage überdies eine den Körper wenigstens teilweise umgebenden Hülle aufweist, die nicht flüssigkeitsabsorbierend ist, und die
a) als Wunddistanzgittter fungiert, oder die
b) eine Wäscheschutzfunktion aufweist.

4. Wundpflegeartikel gemäß einem der vorherigen Ansprüche, **dadurch gekennzeichnet, dass** die Hülle (2; 12; 22) aus einem Material in Sandwichanordnung besteht.

5. Wundpflegeartikel gemäß Anspruch 4, **dadurch gekennzeichnet, dass** das Material in Sandwichanordnung eine Matte vom Typ Airlaid ist.

6. Wundpflegeartikel gemäß einem der vorhergehenden Ansprüche, **dadurch gekennzeichnet, dass** der absorbierende Körper (1; 11; 21) und/oder die Hülle (2; 12; 22) superabsorbierende Polymere (20) aufweist.

7. Wundpflegeartikel gemäß einem der vorherigen Ansprüche, **dadurch gekennzeichnet, dass** die superabsorbierenden Polymere in Form eines Granulats, eines Pulvers, einer Schüttung, eines Presslings, eines Schaums, in Form von Fasern, eines Fasergewirkes, - geleges oder -vlieses und/oder einer Faserwatte vorliegen.

8. Wundpflegeartikel gemäß einem der vorhergehenden Ansprüche, **dadurch gekennzeichnet, dass** wenigstens die Hülle (22) mit nutritiven Zusätzen und/oder Antibiotika für die topische Anwendung bei der Wundheilung versehen ist.

9. Wundpflegeartikel gemäß einem der vorhergehenden Ansprüche, **dadurch gekennzeichnet, dass** der innerhalb der Hülle angeordnete absorbierende Körper (1; 11; 21) ein Airlaid mit hydroaktiven, bevorzugt superabsorbierenden Polymeren in Pulver- oder Granulatform aufweist, während die Hülle hydroaktive, bevorzugt superabsorbierende, Polymere in Faserform aufweist.

10. Wundpflegeartikel gemäß einem der vorhergehenden Ansprüche, **dadurch gekennzeichnet, dass** sowohl die Hülle als auch der innerhalb der Hülle angeordnete absorbierende Körper (1; 11; 21) Airlaid-Material mit darin angeordneten hydroaktiven, bevorzugt superabsorbierenden Polymeren, aufweisen.

11. Wundpflegeartikel gemäß einem der vorhergehenden Ansprüche, **dadurch gekennzeichnet, dass** die Hülle aus einem Schaumstoff und der innerhalb der Hülle angeordnete absorbierende Körper (1; 11; 21) Airlaid-Material mit darin angeordneten hydroaktiven, bevorzugt superabsorbierenden Polymeren aufweisen.

12. Wundpflegeartikel gemäß einem der vorhergehenden Ansprüche, **dadurch gekennzeichnet, dass** sowohl die Hülle als auch der innerhalb der Hülle angeordnete absorbierende Körper (1; 11; 21) Airlaid-Material mit darin angeordneten hydroaktiven, bevorzugt superabsorbierenden Polymeren aufweisen.

13. Wundpflegeartikel gemäß einem der vorhergehenden Ansprüche, **dadurch gekennzeichnet, dass** der innerhalb der Hülle angeordnete absorbierende Körper (1, 11; 21) ein Airlaid mit superabsorbierenden Polymeren in Pulver- oder Granulatform aufweist, während die Hülle superabsorbierende Polymere in Faserform aufweist.

14. Wundpflegeartikel gemäß einem der vorhergehenden Ansprüche, **dadurch gekennzeichnet, dass** die Hülle in Form einer mindestens einseitig offenen, ggf. verschließbaren Tasche ausgebildet ist.

15. Wundpflegeartikel gemäß Anspruch 14, **dadurch gekennzeichnet, dass** die Hülle zumindest abschnittsweise eine luftdichte Schicht sowie mindestens eine Einrichtung zum Anschluß einer Absaugeinrichtung aufweist.

16. Verwendung des Wundpflegeartikels gemäß einem der vorherigen Ansprüche bei einem Kompressionssystem (50) oder einem Unterdrucksystem (70).

## Claims

1. Wound care article (100; 200; 300; 400; 500) comprising at least one exudate absorbing body (1; 11; 21) and at least one shell (2; 12; 22) at least partially surrounding the body (1; 11; 21),
**characterized in that**,
the shell (2; 12; 22) is itself liquid absorbing and the shell comprises flexible foam, preferably polyurethan-foam,
wherein the liquid absorbing body is freely arranged within the shell.

2. Wound care article according to claim 1, **characterized in that** the wound care article is in an unused condition flat or approximately cylindrical.

3. Wound care article according to any of the preceding claims, **characterized in that** the wound care article comprises an additional shell at least partially surrounding the body, wherein the additional shell is not liquid absorbing and this shell
a) acts as a wound distance grid, or
b) comprises a clothing protection function.

4. Wound care article according to any of the preceding claims, **characterized in that** the shell (2; 12; 22) consists of a material comprising a sandwich-type structure.

5. Wound care article according to claim 4, **characterized in that** the material comprising a sandwich-type structure is a mat of airlaid-type.

6. Wound care article according to any of the preceding claims, **characterized in that** the absorbing body (1; 11; 21) and / or the shell (2; 12; 22) comprises super-absorbing polymers.

7. Wound care article according to any of the preceding claims, **characterized in that** the super-absorbing polymers are present in the form of a granulate material, a powder, a ballast, a pellet, a foam, in the form of fibres, a fibre-knitted fabric, -clutch or mat and / or fibre-wadding.

8. Wound care article according to any of the preceding claims, **characterized in that** at least the shell (22) comprises nutritional additives and / or antibiotics for topical application during wound healing.

9. Wound care article according to any of the preceding claims, **characterized in that** the absorbing body (1; 11; 21) arranged within the shell comprises an airlaid including hydro-active, preferably super-absorbing polymers in powder or granular form, wherein the shell comprises hydro-active, preferably super-absorbing polymers in fibre-form.

10. Wound care article according to any of the preceding claims, **characterized in that** the shell as well as the absorbing body (1; 11; 21) arranged within the shell comprise airlaid material, wherein the airlaid material comprises hydro-active, preferably super-absorbing polymers arranged within the airlaid material.

11. Wound care article according to any of the preceding claims, **characterized in that** the shell comprises a foam material and the absorbing body (1; 11; 21) arranged within the shell comprises airlaid material, wherein the airlaid material comprise hydro-active, preferably super-absorbing polymers arranged within the airlaid material.

12. Wound care article according to any of the preceding claims, **characterized in that** the shell as well as the absorbing body (1; 11; 21) arranged within the shell comprise airlaid material, wherein the airlaid material comprises hydro-active, preferably super-absorbing polymers arranged within the airlaid material.

13. Wound care article according to any of the preceding claims, **characterized in that** the absorbing body (1; 11; 21) arranged within the shell comprises an airlaid including super-absorbing polymers in powder- or granular form, and the shell comprises super-absorbing polymers in fibre-form.

14. Wound care article according to any of the preceding claims, **characterized in that** the shell is formed as a bag, wherein the bag is at least open at one side and, if applicable, closable.

15. Wound care article according to claim 14, **characterized in that** the shell comprises at least partially an airtight layer and at least one fitting being connectable to a suction unit.

16. Use of the wound care article according to any of the preceding claims in a compression system (50) or a low pressure system (70).

## Revendications

1. Article de soin de plaies (100 ; 200 ; 300 ; 400 ; 500), contenant au moins un corps absorbant des liquide de plaies (1 ; 11 ; 21) et au moins une enveloppe (2 ; 12 ; 22) entourant au moins partiellement le corps (1 ; 11 ; 21),
**caractérisé en ce que**
l'enveloppe (2 ; 12 ; 22) elle-même absorbe du liquide et l'enveloppe contient de la mousse molle, de préférence de la mousse de polyuréthane,
le corps absorbant étant disposé en vrac à l'intérieur de l'enveloppe.

2. Article de soin de plaies selon la revendication 1, **caractérisé en ce que** l'article de soin de plaies, à l'état non utilisé, forme membrane ou est approximativement cylindrique.

3. Article de soin de plaies selon une des revendications précédentes, **caractérisé en ce que** le recouvrement de plaie contient en outre une enveloppe entourant au moins partiellement le corps et n'absorbant pas du liquide, et qui
a) fait office de grillage d'écartement de la plaie, ou qui
b) a une fonction de protection de linge.

4. Article de soin de plaies selon une des revendications précédentes, **caractérisé en ce que** l'enveloppe (2 ; 12 ; 22) consiste en un matériau disposé en sandwich.

5. Article de soin de plaies selon la revendication 4, **caractérisé en ce que** le matériau disposé en sandwich est une natte de type Airlaid.

6. Article de soin de plaies selon une des revendications précédentes, **caractérisé en ce que** le corps absorbant (1 ; 11 ; 21) et/ou l'enveloppe (2 ; 12 ; 22) contient des polymères (20) super-absorbants.

7. Article de soin de plaies selon une des revendications précédentes, **caractérisé en ce que** les polymères super-absorbants se présentent sous forme de granulé, de poudre, de vrac, de pastille, de mousse, sous forme de fibres, de textile, nappe ou non-tissé fibreux / fibreuse et/ou d'une ouate fibreuse.

8. Article de soin de plaies selon une des revendications précédentes, **caractérisé en ce qu'**au moins l'enveloppe (22) est pourvue d'additifs nutritifs et/ou d'antibiotiques pour l'application topique lors de la cicatrisation de plaies.

9. Article de soin de plaies selon une des revendications précédentes, **caractérisé en ce que** le corps absorbant (1 ; 11 ; 21) disposé à l'intérieur de l'enveloppe contient un matériau fabriqué par voie sèche (Airlaid) contenant des polymères hydroactifs, de préférence super-absorbants, sous forme de poudre ou de granulés, tandis que l'enveloppe contient des polymères hydroactifs, de préférence super-absorbants, sous forme de fibres.

10. Article de soin de plaies selon une des revendications précédentes, **caractérisé en ce qu'**aussi bien l'enveloppe que le corps absorbant (1 ; 11 ; 21) disposé à l'intérieur de l'enveloppe contiennent du matériau Airlaid ayant agencés là-dedans des polymères hydroactifs, de préférence super-absorbants.

11. Article de soin de plaies selon une des revendications précédentes, **caractérisé en ce que** l'enveloppe en une mousse et le corps absorbant (1 ; 11 ; 21) disposé à l'intérieur de l'enveloppe contiennent du matériau Airlaid ayant agencés là-dedans des polymères hydroactifs, de préférence super-absorbants.

12. Article de soin de plaies selon une des revendications précédentes, **caractérisé en ce qu'**aussi bien l'enveloppe que le corps absorbant (1 ; 11 ; 21) disposé à l'intérieur de l'enveloppe contiennent du matériau Airlaid ayant agencés là-dedans des polymères hydroactifs, de préférence super-absorbants.

13. Article de soin de plaies selon une des revendications précédentes, **caractérisé en ce qu'**aussi bien l'enveloppe que le corps absorbant (1 ; 11 ; 21) disposé à l'intérieur de l'enveloppe contient du matériau Airlaid ayant des polymères super-absorbants sous forme de poudre ou de granulés, tandis que l'enveloppe contient des polymères super-absorbants sous forme de fibres.

14. Article de soin de plaies selon une des revendications précédentes, **caractérisé en ce que** l'enveloppe est réalisée sous forme d'au moins une poche ouverte d'au moins un côté, éventuellement refermable.

15. Article de soin de plaies selon la revendication 14, **caractérisé en ce que** l'enveloppe contient du moins par endroits une couche hermétique à l'air et au moins un dispositif pour le raccordement d'un dispositif d'aspiration.

16. Article de soin de plaies selon une des revendications précédentes dans un système de compression (50) ou un système de dépression (70).
